# EUROPÄISCHE PATENTSCHRIFT

(11) **EP 2 928 834 B1**
(45) Veröffentlichungstag und Bekanntmachung des Hinweises auf die Patenterteilung: **08.11.2017**
(21) Anmeldenummer: 13805767.4
(22) Anmeldetag: 27.11.2013
(51) Int. Cl.: C02F 3/28, C02F 11/04, C12M 1/00, C12M 1/107, C12P 5/02

(54) **VERFAHREN UND EINRICHTUNG ZUM BETRIEB EINER BIOGASANLAGE MIT AQUATISCHEN PFLANZEN**
METHOD AND APPARATUS FOR OPERATING A BIOGAS SYSTEM USING AQUATIC PLANTS
PROCEDE ET DISPOSITIF POUR FAIRE FONCTIONNER UNE INSTALLATION DE PRODUCTION DE BIOGAZ AVEC DES PLANTES AQUATIQUES

(30) Priorität: 10.12.2012 DE 102012024108
(43) Veröffentlichungstag der Anmeldung: 14.10.2015
(73) Patentinhaber: Rogmans, Maria, 47546 Kalkar (DE)
(72) Erfinder: WILHELM, Hermann-Josef, 47546 Kalkar (DE)
(74) Vertreter: Schmidt, Karl Michael
(86) Internationale Anmeldenummer: PCT/EP2013/003578
(87) Internationale Veröffentlichungsnummer: WO 2014/090376

(56) Entgegenhaltungen:
- EP-A1- 0 484 867
- WO-A2-2009/090476
- DE-A1-102008 050 974
- DE-A1-102011 012 446
- GB-A- 2 484 530
- NL-C2- 2 001 244
- US-A1- 2010 105 127
- US-A1- 2012 308 989
- CLARK P B ET AL: "Enhancement of anaerobic digestion using duckweed (Lemna minor) enriched with iron", JOURNAL OF THE CHARTERED INSTITUTION OF WATER AND ENVIRONMENTAL MANAGEMENT 199604 GB, Bd. 10, Nr. 2, April 1996 (1996-04), Seiten 92-95, XP002724966, ISSN: 1360-4015
- JAIN S K ET AL: "PRODUCTION OF BIOGAS FROM AZOLLA-PINNATA R.BR. AND LEMNA-MINOR L. EFFECT OF HEAVY METAL CONTAMINATION", BIORESOURCE TECHNOLOGY, Bd. 41, Nr. 3, 1992, Seiten 273-277, XP002724967, ISSN: 0960-8524
- TRISCARI P ET AL.: "Anaerobic digestion of dairy manure combined with duckweed (Lemnaceae)", AMERICAN SOCIETY OF AGRICULTURAL AND BIOLOGICAL ENGINEERS ANNUAL INTERNATIONAL MEETING 2009, RENO, NV, Bd. 2, 095765, 21. Juni 2009 (2009-06-21), - 24. Juni 2009 (2009-06-24), Seiten 980-989, XP002724968,
- SCHMIDT K. M. ET AL.: "Steigerung der Effektivität in derMethanproduktion bei Biogasanlagen mit Hilfe einer zeitlich koordinierten Fütterung eines sauren Biogas-Boosters bei parallelem Einsatz eines H2S-Binders sowie deren mathematischeBeschreibung", AGROPHYSICAL LETTERS, April 2016 (2016-04), pages 129-150,

## Beschreibung

Die Erfindung betrifft ein Verfahren zum Betrieb einer Biogasanlage bei welcher landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in einem Gärprozess vergärt oder in einem weiteren Gärprozess nachvergärt wird, gemäß Oberbegriff des Patentanspruchs 1.

Biogasanlagen mit aquatischen Pflanzen als Gärsubstrat zu betreiben ist als solches aus der DE 2008 050 974 A1 vom selben Anmelder bekannt.
Dabei werden die aquatischen Pflanzen in gestapelten Wannensystemen kultiviert und werden als Gärsubstrat zur Biogaserzeugung herangezogen.
Dabei hat sich aber auch gezeigt, dass der ausschließliche Betrieb einer solchen Kultureinrichtung nur zur Biogaserzeugung wegen des benötigten Wärmehaushaltes und der baulichen Maßnahmen in wirtschaftlicher Hinsicht kritisch sein könnte.
Somit macht es Sinn, dieselbe Kultureinrichtung zeitgleich mit der Biomassenpropagation noch einem weiteren Zweck, nämlich dem der Abwasserbehandlung zuzuführen.

Aus der DE 10 2011 012446 A1ist ein Schichtkomposter mit einer sogenannten Trockenfermentation bekannt. Dabei werden auch aquatische Pflanzen mit kompostiert.
Aus der US 2012/308989 A1 ist ein Fermentationsverfahren bekannt, bei welchem aquatische Pflanzen bspw mit Gülle vermischt in einem Biogasprozess fermentiert werden können.
Insgesamt gelten aquatische Pflanzen, insbesondere Lemnaceae und Azolla als leicht vergärbar in Biogasprozessen.

Die Verwertung von Gülle in Biogasanlagen ist ebenfalls bekannt. Dabei ist es auch bekannt Gülle entweder zusätzlich, bspw als Impfgülle einzusetzen, oder die Biogasanlage auf ausschließlich Gülle als Gärsubstrat zu betreiben.

Nachteilig ist dabei, dass die ausgegasten Gärreste wieder auf Ackerflächen ausgebracht werden müssen, was mit Zeit- und Kostenaufwand verbunden ist und außerdem zur Eutrophierung führen kann.

Der Erfindung liegt daher die Aufgabe zugrunde, ein Verfahren sowie eine Einrichtung der gattungsgemäßen Art dahingegehend weiter zu entwickeln, dass beim Einsatz von landwirtschaftlicher Gülle, insbesondere der Gülle aus der Viehhaltung aber auch beim Einsatz bereits vorgegaster oder teilausgegaster Gärreste aus herkömmlichen Biosubstraten von Biogasanlagen ein zusätzlicher Biogasertrag erzielbar ist, und dass eine Ausbringung von finalen Gärresten auf Ackerflächen vermieden oder erheblich reduziert wird.

Kern der verfahrensgemäßen Erfindung ist, dass aus der Gülle oder dem zuvor teilausgegasten Gärbrei oder Klärschlamm in einem ersten Schritt die Dickphase von der Dünnphase abgeschieden wird, dass die Dickphase in den Gärraum der Biogasanlage eingebracht wird, dass die abgeschiedene Dünnphase einer Kultureinrichtung mit schnellwüchsigen aquatischen Pflanzen zugeführt wird, und zumindest ein Teil der aus der Kultureinrichtung regelmäßig entnehmbaren Biomasse aus aquatischen Pflanzen dem Gärraum der mit der Dickphase befüllten Biogasanlage zugeführt wird.

Dadurch entsteht ein intelligenter Massenstrom, bei dem auch die flüssigen Gärreste oder die Dünnphase aus Gülle zeitnah, d.h. quasi in-situ wieder in extrem schnellwüchsige aquatische Biomasse konvertiert wird, und so dem Biogasprozess wieder in einem oder wenigen Tagen zugeführt werden kann.

Im Ergebnis führt dies zu folgenden ganz erheblichen Vorteilen:
1. Zunächst scheinbar ausgegastes Gärsubstrat aus Biogasanlagen, die mit beliebiger Biomasse befüttert wurden, wird auf diese Weise wieder belebt. Die Trennung in zwei Massenströme Dünnphase und Dickphase nimmt im Vorgang der biogastechnischen Neubelebung zusätzlich noch die quasi im Nebenstrom erzeugbare Erzeugung eines zusätzlichen Biogasboosters (Biogasverstärker) mit.
2. Gülle aus der Viehhaltung wird in gleicher Weise behandelt, und erzeugt duch die Aufteilung in zwei Massenströme das belebte Biogasgärsubstrat der Dickphase einerseits und durch die mit der Dünnphase generierte aquatische Biomasse die Erzeugung eines Biogasboosters noch mit.
3. Durch den zyklischen Betrieb wird zum einen die anfallende Gülle, samt sonstig anfallenden Gärresten aus der Biogasproduktion vollständig entsorgt.
   Zum anderen entsteht ein geschlossener Stoffkreislauf mit einem erheblichen zusätzlichen Energieertrag aus Biogas.
4. Auch Klärschlämme aus Wasserkläranlagen können auf dieselbe Weise behandelt werden.

In weiterer vorteilhafter Ausgestaltung ist angegeben, dass als schnellwüchsige aquatische Pflanzen Wasserlinsen oder Algen in der Kultureinrichtung produziert werden. Dies hat zur Folge, dass der quasi rückdüngende Beitrag aus der Dünnphase mit nur einem Tag oder weniger Zeitversatz schon aquatische Biomasse daraus erzeugt. Dies liegt daran, dass in den genannten Kultureinrichtungen für aquatische Pflanzen, insbesondere Wasserlinsen der dadurch erzielte Biomassenertrag nach einem Tag schon bereit steht. Im Vergleich dazu dauert beim herkömmlichen Verfahren mit bspw Mais als Biomassensubstrat für Biogasanlagen ab Rückdüngung die nächste Ernte mehr als 9 Monate.

Demgegenüber kann mit Hilfe der genannten aquatischen Kultureinrichtung insbesondere mit Wasserlinsen im Gegensatz zu bspw Mais überhaupt erst ein tatsächliches in-situ-Verfahren wegen der täglichen Biomassenvermehrung von Wasserlinsen und anderen extrem schnellwüchsigen aquatischen Pflanzen vorgenommen werden.

In weiterer vorteilhafter Ausgestaltung ist daher angegeben, dass der Biomasse aus aquatischen Pflanzen ein Biogas-Booster vor oder mit Einbringung in den Gärraum einer Biogasanlage zugemischt wird, wobei der Biogas-Booster aus einem oder mehreren Fettsäureester bzw Fettsäureestern oder Fetten oder Ölen, und/oder Schlempen, und/oder fetthaltigen oder Zucker- oder Alkoholspuren oder Hefen enthaltenden Resten aus der Lebensmittelproduktion, sowie aus Spurenelementen besteht.

Mit Hilfe dieses Biogas-Booster sind die oben genannten hohen zusätzlichen Biogasausbeuten erzielbar. Außerdem erfolgt der Biogasaufschluss auch so schnell, dass dies der extrem schnellen Verfügbarkeit der besagten aquatischen Pflanzen Rechnung trägt.

Das besagte Verfahren kann vorteilhaft über einen ganz erheblichen Mischungsbereich gefahren werden. Diese ist so benannt, dass der Anteil der Mischung aus aquatischen Pflanzen plus Biogasbooster im Gärbrei der Biogasanlage in Summe bei ca 2% bis 90% der Feuchtmasse des Gärbreies der im Gärraum eingebrachten Dickphase liegt. Die Wahl dieses weiten Bereiches basiert darauf, dass die oben genannten funktionalen Konsequenzen auf erfindungsgemäße Weise miteinander in Wirkung gebracht werden können.
Der hohe und extrem schnelle Massenzuwachs und die Stoffzusammensetzung des Biogas-Boosters ermöglichen das ertragreiche Fahren dieses weiten Mischungsbereiches erst. Das Besondere liegt hierbei also in dem möglichen weiten Mischungsbereich, in welchem die genannten Vorteile erzielbar sind.

In einer Ausgestaltung kann es von erheblichem Vorteil sein, dass die Biogasanlage in oder direkt neben der Kultureinrichtung für aquatische Pflanzen angeordnet wird, und dass die Wärmekreisläufe zusammengeschaltet sind, derart, dass die Kultureinrichtung zumindest teilweise von der Abwärme der Biogasanlage beheizt wird. Dies gilt insbesondere dann, wenn man eine Vielzahl dezentraler Einrichtungen plant.

Aber auch bei der Zentralisierung jeweils der Biogasanlage zum einen, und der aquatischen Kultureinrichtung zum anderen kann dies seine Vorteile in genossenschaftlicher Betriebsweise ebenso nachhaltig entfalten.

In weiterer vorteilhafter Ausgestaltung ist angegeben, dass die Dickphase pyrolisiert, oder in einem sonstigen Verkohlungsschritt durch hydrothermale Karbonisierung oder durch Trockenfermentationsprozesse eines quasi aufkohlenden Kompostierverfahrens behandelt wird.

Es ist eine Kultureinrichtung angegeben, in welcher der Biogasanlage eine Kultureinrichtung für aquatische Pflanzen beigeordnet wird, und dass eine Trenneinrichtung zur physischen Trennung einer Dickphase und einer Dünnphase aus landwirtschaftlicher Gülle, oder vorvergastem Gärsubstrat vorgesehen ist, sowie eine Transport- und/oder Mischeinrichtung zur zusätzlichen Befüllung der Biogasanlage mit aquatischen Pflanzen oder mit einem aus aquatischen Pflanzen und einem Biogashilfsstoff erstellten Zusatzsubstrat.
Auch dies betrifft sowohl den Einsatz von Gülle aus der Viehhaltung als auch die Reste aus der Biogasproduktion.

Weitererhin ist beschrieben, dass die Mischeinrichtung eine geregelte Mischeinrichtung ist, und auf der Basis der eingegebenen Eingangsstoffe ein definiertes reproduzierbares Mischungsverhältnis erstellt. Auch wenn der Bereich des Mischungsverhältnisses, wie oben ausgeführt, weit gefasst ist, kommt der Reproduzierbarkeit im Anlagenbetrieb dennoch eine wesentliche Rolle bei der gesteuerten und ertragsmaximierten Biogasproduktion zu.Der gewählte weite Mischungsbereich erlaubt es, bei der Auslegung der aquatischen Kultureinrichtung im Massenertrag einen Anteil von 2% bis 90% zu planen. D.h. dass Anlagenkonzepte, bei denen die Kultureinrichtung 2% zum Biogassubstrat beiträgt, die Gesamtkapazität der zugehörigen Biogasanlage im Massendurchsatz 50 mal größer sein kann, weil ja 98 % Fremdsubstrat und nur 2% Substrat aus der besagten aquatischen Kultureinrichtung beigesteuert ist. Dennoch wird dann eine solche Anlage im Mischer genau auf diesem Wert gefahren.
Bei einer 50%/50% - Anlage oder einer 90%/10% - Anlage in entsprechender Weise.

Die Befüllung der Biogasanlage über automatische Füllmittel erfolgt, die den gemischten Biogashilfsstoff in definierter reproduzierbarem Mengenverhältnis zum vorhanden Gärbrei zumischt.

Die Kultureinrichtung ist für die aquatischen Pflanzen im Bodenbereich aus einem aus systemsteigenen zusammengestellten Fundament bestehend, auf welchem ein Etagensystem von Kulturwasserwannen aufgesetzt ist.
Zu Systemsteinen gehören Steine, mit komplementärer Außenkontur oder mit komplementären Außenstrukturen, in denen beim Zusammenbau Ausbuchtungen des jeweils einen Steins in Einbuchtungen des jeweils anderen Steins eingreifen und so eine definierte Lageposition vorgeben. So entsteht am Ende ein kraft- und teilförmschlüssiger Verbund. So lassen sich Fundamente bauen, ohne die ansonsten üblichen Betonfundamentarbeiten vornehmen zu müssen. Somit sind Kultureinrichtungen dieser Art wesentlich einfacher , schneller und kostengünstiger zu errichten.

Die Kultureinrichtung ist mit einer geschlossenen oder schließbaren Folieneinhausung versehen ist, in welche hohe Konzentrationen von CO2 einleitbar sind. Außerdem ist die geschlossene Bauform auch für das oben dargestellte schlüssige Wärmekonzept wichtig.
Um die oben bereits beschriebenen internen Stoffströme auch im Hinblick auf den Wasserhaushalt durchführen zu können ist beschrieben,dass die Folieneinhausung zumindest in Bezug auf den vertikalen Querschnitt mit einer steten Krümmung versehen ist, und unten mit Auffangrinnen zum Auffangen des kondensierten Verdunstungswassers versehen oder verbunden ist. So sind im Temperaturwechsel im Tag-Nacht-Rhythmus oder einfach durch eine kühlere Aussenhaut die entstehenden Verdunstungsmengen, die bei gestapelten aquatischen Wannenkulturen entstehen, einfach zurückzuführen.
Die Kultureinrichtung weist bodenseitig eine im oder mit dem Fundament eingelassene Bodenwanne auf, in welche ein Wasservorlauf für das Kulturwasser erstellt wird. Auf diese Weise ist ein Stoffpuffer vorhanden, mit dem Fluktuationen im Anfall von Dünnphase aus der landwirtschaftlichen Gülle ausgeglichen werden können, und sattdessen ein im weiteren dennoch konstanter Stoffstrom erzeugt werden kann.

Die aquatischen Pflanzen können getrocknet, angetrocknet oder als Frischmasse mit dem Biogas-Booster. In weiterer vorteilhafter Ausgestaltung ist vorgesehen, dass die aquatischen Pflanzen getrocknet, angetrocknet oder als Frischmasse zugeführt sind. Dies umfasst mehrere Alternativen. Verwendet man zur Herstellung des Biogashilfsstoffes die aquatischen Pflanzen, bspw Wasserlinsen im getrockneten Zustand, dann nehmen sie aufgrund ihrer offenporigen Textur maximal viel der übrigen zumindest teilweise flüssigen Komponenten des Biogasboosters auf, bevor sie dann dem Massenstrom zum Biogasfermenter zugegeben werden. Da aquatische Pflanzen, insbesondere Wasserlinsen einen Trockensubstanzgehalt von ca 10% haben, also im frischen Zustand aus 90 % Wasser bestehen, welches natürlich im getrockneten Zustand verdunstet ist, so hat diese Biomasse eine dem ursprünglichen Wassergehalt nahezu entsprechende Aufnahmefähigkeit für die Boosterstoffe, die der verdunsteten Wassermenge nahezu entspricht. Wasserlinsen haben eine starke Gasbildungsrate und vor allem gasen sie schnell. Auf diese Weise wird ein biogener Trägerstoff für die Boosterstoffe bereitgestellt, der bei Biogaserzeugung mit fermentiertund somit alle Boosterstoffe mit freisetzt. Man kann sie aber auch getrocknet, bspw mit einem Trockensubstanzgehalt von ca 35% verwenden.
Verwendet man die aquatischen Pflanzen als Frischmasse, dann haben sie einen Trockensubstanzgehalt der in etwa dem Trockensubstanzgehalt des Gärbreis in Biogasanlagen entspricht. Diese Variante ist somit auch vorteilhaft.

Im Hinblick auf ein Verfahren zur Herstellung eines Biogashilfsstoffes ist erfindungsgemäß vorgeschlagen, dass die aquatischen Pflanzen getrocknet, oder angetrocknet oder als Frischmasse mit dem Biogas-Booster, welcher wiederum aus einem oder mehreren Fettsäureester bzw Fettsäureestern oder Fetten oder Ölen, und/oder Schlempen, und/oder fetthaltigen oder Zucker- oder Alkoholspuren oder Hefen enthaltenden Resten aus der Lebensmittel-produktion, sowie aus Spurenelementen besteht, vermischt wird bzw werden.
Dies führt zu den oben bereits dargestellten Vorteilen.

Ein Ausgestaltungsbeispiel der Erfindung ist in der Zeichnung dargestellt und nachfolgend näher beschrieben.

Es zeigt:
- Figur 1:: Stoffströme im erfindungsgemäßen Verfahren
- Figur 2:: Ausgestaltung einer Kultureinrichtung
- Figur 3:: Stoffströme in alternativen Betriebsweisen

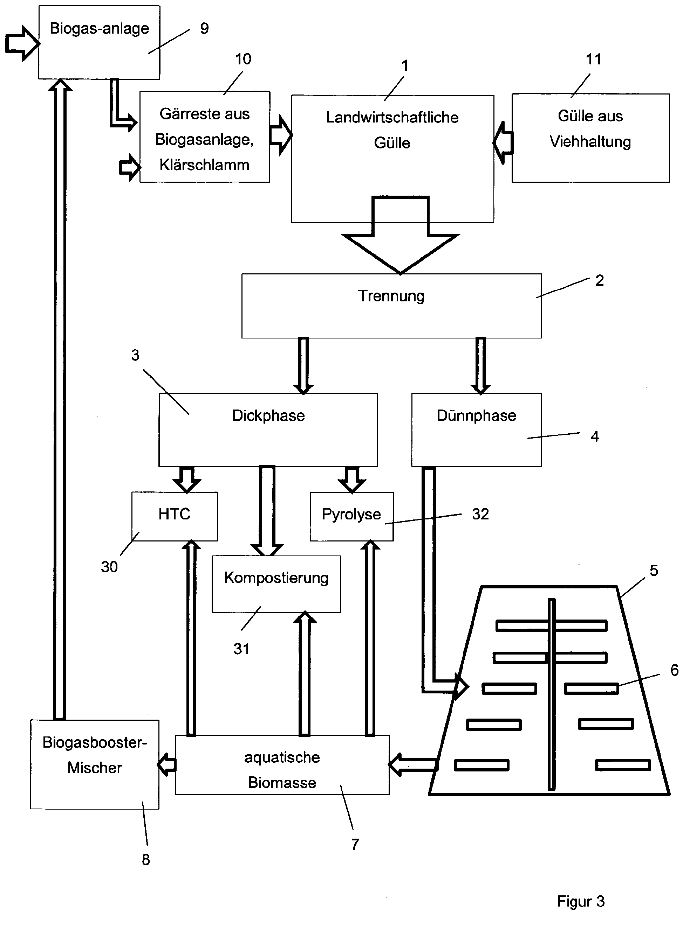

Figur 1 zeigt die Stoffströme im erfindungsgemäßen Verfahren. Hierbei wird unter landwirtschaftliche Gülle 1 sowohl die Gülle aus der Viehhaltung verstanden, als auch die Gärreste/Gärsäfte aus Biogasanlagen 9, sogar Klärschlämme aus Wasserkläranlagen, oder sogar Prozessabwasser aus der Lebensmittelproduktion mit umfasst.

Die Darstellung besagt aber nicht, dass alle verschiedenen Güllen zusammengeführt würden, oder werden müssten, sondern jede dieser genannten einzelnen Güllen sich für die Anwendung dieses erfindungsgemäßen Verfahrens eignet. Eine Güllemischung ist aber dennoch nicht ausgeschlossen. Sie ist nur nicht obligatorisch.

Die landwirtschaftliche Gülle 1 wird in Dünnphase 4 und Dickphase 3 getrennt. Zumeist sedimentiert die Dickphase innerhalb der Dünnphase, so dass die Trennung leicht ist. Dann teilt sich der so erhaltene Stoffstrom. Die Dünnphase 4 wird dem Kulturwasser von aquatischen Kulturpflanzen entweder direkt zugeführt, d.h. dem Kulturwasser beigemischt, oder es wird zuvor noch eine Fällung vorgenommen oder es wird durch die Einbringung von Luft-Sauerstoff der Stickstoff gasförmig ausgetrieben. Dies kann sogar mit entsprechenden Bakterien erzielt werden. Sodann wird bei dieser Reaktion Luft-Stickstoff frei, so dass zweckmäßigerweise die gefällte Dünnphase in der aquatischen Kultureinrichtugn zuerst einer Kultur aus Leguminosen oder einer aquatischen Kultur von Azolla zugeführt. Diese führen eine biogene Verstoffwechselung des entstehenden Stickstoffs durch. Zur effektiven Verstoffwechselung des möglichst gesamt anfallenen Luft-Stickstoffs kann dabei die Azolla- oder Leguminosen-Kultur in einem gasdicht abgeschlossenen Untercompartment der aquatischen Kultureinrichtung mit integriert sein. Hernach kann das Kulturwasser dann bspw Kulturen mit Wasserlinsen oder anderen aquatischen Pflanzen weitergeschleust werden. Vorzugsweise sind die Kulturwannen 6 der Kultureinrichtung 5, in denen die aquatischen Kulturpflanzen kultiviert werden, gestapelt, und es sind zumindest teilweise Schwachlichtpflanzen, wie Wasserlinsen dort verwendet.
Inbesondere Wasserlinsen populieren extrem schnellwüchsig mit bis zu einer Verdopplung pro Tag.
Dies führt wiederum dazu, dass dieses Stoffstromsystem innerhalb nur eines Tages auf die Dünnphasezufuhr bereits wuchsbeschleunigend reagiert. Die im Kultursystem bspw durch Floating erzielbare Erntemenge an aquatischer Biomasse 7 wird sodann einem Biogasbooster-Mischer 8 zugeführt. Von dort wird das Gemisch aus aquatischen Pflanzen und Biogas-Booster sodann der Biogasanlage 9 zugeführt, in der auch übrige fremde Biomasse vergärt wird, die nicht aus diesem Prozess stammt.

Der Biogasbooster besteht somit aus aquatischen Pflanzen zum einen und aus weiteren oben genannten Komponenten zum anderen.

Die dann bei der Biogasproduktion wieder entstehenden Gärreste werden wieder entnommen und getrennt nach Dickphase und Dünnphase, wobei die Dickphase der Biogasanlage 9 direkt wieder zurückgeführt wird. Spätestens hier können dann Gärreste und neue Gülle ggfs gemischt und dem Kulturwasser der aquatischen Kultureinrichtung zugeführt werden.

Wichtig ist hierbei, dass im Gärbrei auch die Dickphase solange im Kreis gefahren wird, bis diese vollständig zersetzt und von der Dünnphase mit in die Verstoffwechselung innerhalb der aquatischen Kultureinrichtung gefahren wird, dort wieder in Biomasse umgewandelt und so wieder als Booster, und/oder gasfähiges Gärsubstrat zur Verfügung steht.

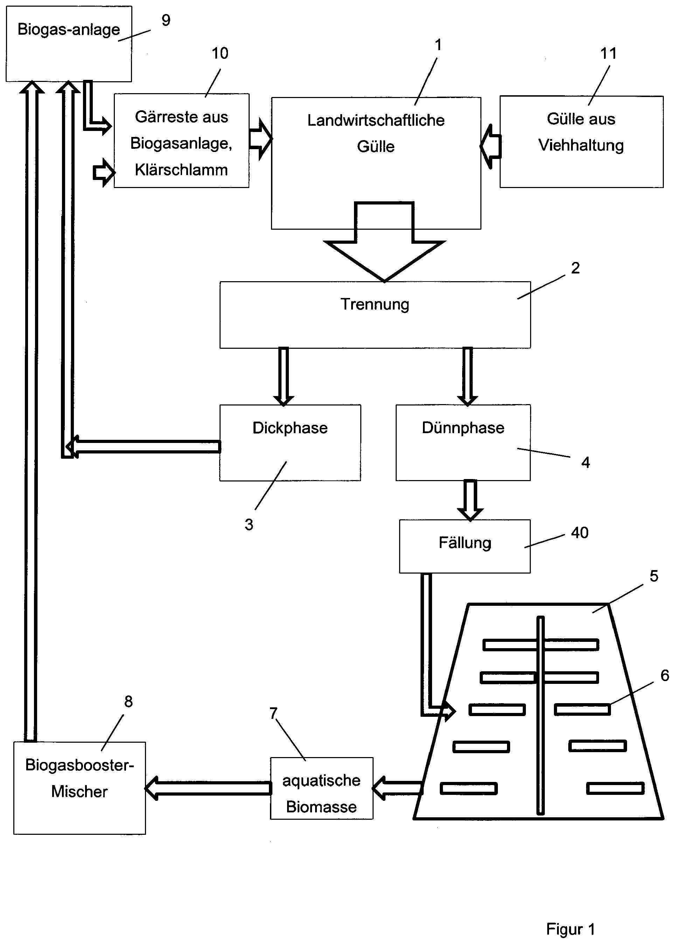

Neben dem Betrieb einer Biogasanlage 9 ist auch der Betrieb eines Komposters alternativ vorgesehen. In diesem kann die Dickphase zusammen mit anderen Substraten bspw sogar in einer Trockenfermentation kompostiert werden. Auch dabei fallen Ablaufsäfte an, die wieder in der gleichen Weise rezykliziert werden können. Hierzu weiter unten zu Figur 3.

Nach der ersten sowie der zweiten Alternative bleibt bei dem erfindungsgemäßen Verfahren keinerlei Rest mehr übrig.

Insgesamt ist zu berücksichtigen, dass alle in dem Prozess enthaltenen Verfahrenstufen, die Abwärme erzeugen, diese in die aquatischen Kultureinrichtung, bspw durch Zuführung zum Kulturwasser eingeleitet wird. Diese Wärme wirkt sich unmittelbar auf einen höheren Biomassenertag aus. Somit entsteht desweiteren ein schlüssiges Wärmekonzept.

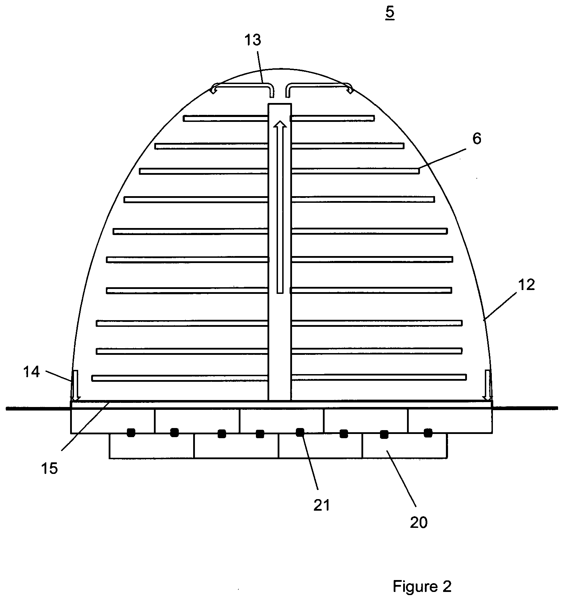

Figur 2 zeigt eine mögliche Ausgestaltungsform einer kompakten und einfachen Kultureinrichtung 5 für aquatische Kulturpflanzen. Zentralelement ist das Regalsystem, in welchem Kulturwasserwannen 6 mit einem für die Lichtverteilung notwendigen Abstand gestapelt sind. Diese Abstände in der Höhe können 0,2 meter bis 0,5 meter sein. Da Wasserlinsen Schwachlichtpflanzen sind, ist die Stapelung hierbei einfach möglich. Das System kann vorzugsweise aber auch Mischkulturen enthalten, so bspw oben im lichtstarken Bereich aufwachsende aquatische Pflanzen, die keine Schwachlichtpflanzen sind, und unten dann Kulturwannen für Schwachlichtpflanzen. Aquatische Pflanzen, die aufschwimmen oder aus der Wasseroberfläche herauskommen, haben naturgemäß relativ hohe Verdunstungsraten.
Das System selbst ist bspw mit einer Folieneinhausung 12 vollständig geschlossen.
So steigt bis zur vollständigen Sättigung der Luftfeuchte die gesättigte Luft auf, bedingt durch den Treibhauseffekt und die damit bewirkte Thermik im Folienbblock 12, und bewirkt an kühlen Flächen, bspw im Nachtzyklus die Kondensation 13, und ein Rücklauf des Kondenswassers 14 über die Folie 12 nach unten in eine Bodenwanne 15. Dort stehen dann große Mengen Kondenswasser an, die als Mischwasser wieder mit der Dünnphase gemischt und wieder den Kulturen rezykliziert werden kann. Somit fährt das Wasser im Kreis. Gleichzeitig werden in kurzen Zeitabständen täglich oder innerhalb weniger Tage zyklisch Biomasse entnommen. Die mit der Biomassenentnahme entnommene Wassermenge in den aquatischen Pflanzen muss durch Fremdwasser wieder ersetzt werden, bspw durch Brunnenwasser. Auch Schleppverluste von Wasser, die durch kurzzeitige Zwangsbelüftungen entstehen können, müssen so ersetzt werden.
Die ersetzten Wassermengen bleiben aber immer kleiner als der durch Kondensation rückgeführte Wasserkörper.

Bei dieser einfachen Bauform der Kultureinrichtung können Folienblöcke verwendet werden. Zur Statik der Stapelsysteme genügt in erfindungsgemäßer Weise, den Boden nicht als Betonplatte auszuführen, sondern mit Systemsteinen 20, die mit komplementären Ausformungen und Einformungen 21 versehen sind, um diese beliebig zusammenzustellen, so dass diese lagefixiert bleiben. Ein solches System ist leicht aufzubauen, kostengünstig, und bei Standortverschiebung auch wieder leicht abzubauen.

Figur 3 zeigt ein Verfahren ähnlich wie in Figur 1, wobei die aus der aquatischen Kultureinrichtung entnommene Biomasse, d.h. geerntete aquatische Pflanzen, nicht oder nicht mehr vollständig zur Biogasproduktion 9 rezykliziert werden, sondern zusammen mit der Dickphase einer Karbonisierung in einem hydrothermalen Karbonisierungsverfahren 20, und/oder einer Trockfermentation 31, d.h. einer anaeroben Kompostierung, und/oder einer normalen Kompostierung, und/oder einer Pyrolyse 32 zugeführt wird.

Dabei können bei diesen Verfahren wieder entstehende Abwässer auch in der aquatischen Kultureinrichtung ins Kulturwasser zugemischt werden.

Ebenso wie die Stoffschlüssigen Kreisläufe, werden auch alle anfallenden Abwärmen in die aquatische Kultureinrichtung wuchsfördernd zugeführt. Damit ist auch hierbei ein schlüssiges Wärmekonzept als weiterer wesentlicher Vorteil gegeben.

### Bezugszeichen

- 1: Landwirtschaftliche Gülle
- 2: Trennung
- 3: Dickphase
- 4: Dünnphase
- 5: aquatische Pflanzenkultureinrichtung
- 6: Kulturwannen
- 7: aquatische Biomasse
- 8: Biogas-Booster-Mischer
- 9: Biogasanlage /Gärraum
- 10: Gärreste aus Biogasanlage / Klärschlamm
- 11: Gülle aus Viehhaltung
- 12: Hülle der Kultureinrichtung
- 13: Aufsteigende Verdunstungsfeuchte
- 14: Kondensatrücklauf
- 15: Bodenwanne
20 Fundamentsteine
21 komplementäre Strukturen in Fundamentsteinen

30 HTC, Hydrothermale Karbonisierung
31 Kompostierung, anaerobe Fermentation
32 Pyrolyse
40 Fällung und/oder Luft-Sauerstoff-Zufuhr

## Patentansprüche

1. Verfahren zum Betrieb einer Biogasanlage bei welcher landwirtschaftliche Gülle und/oder in einem vorherigen Gärprozess bereits teilausgegaster Gärbrei, und/oder Klärschlamm, und/oder Abfälle aus der Lebensmittelproduktion als Gärssubstrat in einem weiteren Gärprozess vergärt oder in einem weiteren Gärprozess nachvergärt wird,
**dadurch gekennzeichnet,**
**dass** aus der Gülle oder dem zuvor teilausgegasten Gärbrei oder Klärschlamm oder den Lebensmittelproduktionsabfällen in einem ersten Schritt die Dickphase von der Dünnphase abgeschieden wird, dass die Dickphase in den Gärraum der Biogasanlage eingebracht wird, dass die abgeschiedene Dünnphase einer Kultureinrichtung mit schnellwüchsigen aquatischen Pflanzen zugeführt wird, und zumindest ein Teil der aus der Kultureinrichtung regelmäßig entnehmbare Biomasseaus aquatischen Pflanzen dem Gärraum der mit der Dickphase befüllten Biogasanlage zugeführt wird, und dass der Biomasse aus aquatischen Pflanzen ein Biogas-Booster vor oder mit Einbringung in den Gärraum einer Biogasanlage zugemischt wird, wobei der Biogas-Booster aus einem oder mehreren Fettsäureester bzw Fettsäureestern oder Fetten oder Ölen, und/oder Schlempen, und/oder fetthaltigen oder Zucker- oder Alkoholspuren oder Hefen enthaltenden Resten aus der Lebensmittelproduktion, sowie aus Spurenelementen besteht.

2. Verfahren nach Anspruch 1,
**dadurch gekennzeichnet,**
**dass** als schnellwüchsige aquatischen Pflanzen Wasserlinsen oder Algen in der Kultureinrichtung produziert werden.

3. Verfahren nach Anspruch 1 oder 2,
**dadurch gekennzeichnet,**
**dass** der Anteil der Mischung aus aquatischen Pflanzen plus Biogasbooster im Gärbrei der Biogasanlage in Summe bei ca 2% bis 90% der Feuchtmasse des Gärbreiesder im Gärraum eingebrachten Dickphase liegt.

4. Verfahren nach einem der vorhergehenden Ansprüche,
**dadurch gekennzeichnet,**
**dass** die Biogasanlage in oder direkt neben der Kultureinrichtung für aquatische angeordnet wird, und dass die Wärmekreisläufe zusammengeschaltet sind, derart, dass die Kultureinrichtung zumindest teilweise von der Abwärme der Biogasanlage beheizt wird.

5. Verfahren nach Anspruch 4,
**dadurch gekennzeichnet,**
**dass** die Dickphase pyrolisiert oder in einem sonstigen Verkohlungsschritt behandelt wird.

6. Verfahren nach einem der vorhergehenden Ansprüche,
**dadurch gekennzeichnet,**
**dass** die Dünnphase vor Einleitung in die aquatische Kultureinrichtung mit Luft-Sauerstoff belüftet und/oder mit einem Fällungsmittel versehen wird, und zunächst einer Kultur aus Leguminosen und/oder Azolla zur Verstoffwechselung des entstehenden Luft-Stickstoffs zugeführt, und von dort dann der aquatischen Kultureinrichtung weitergeleitet wird.

## Claims

1. Process for operating a biogas plant, in which agricultural slurry and/or fermentation mash which has already partially been degassed in a previous fermentation process, and/or sewage sludge, and/or waste from food production, being the fermentation substrate, is fermented in a further fermentation process or post-fermented in a further fermentation process,
**characterized in that,**
in a first step, the thick phase is separated from the thin phase of the slurry or the previously partially degassed fermentation mash or the sewage sludge or the food production waste, **in that** the thick phase is introduced into the fermentation chamber of the biogas plant, that the thin phase which has been separated is fed to a culture unit which includes rapidly growing aquatic plants, and at least part of the biomass of aquatic plants, which can regularly be removed from the culture unit, is fed to the fermentation chamber of the biogas plant which is filled with the thick phase, and that a biogas booster is admixed to the biomass of aquatic plants before or together with the introduction into the fermentation chamber of a biogas plant, where the biogas booster consists of one or more fatty acids ester(s) or fats or oils, and/or vinasses, and/or food production residues containing fats or containing traces of sugar or alcohol or containing yeast, and of trace elements.

2. Process according to Claim 1,
**characterized in that**
the rapidly growing aquatic plants produced in the culture unit are duckweed or algae.

3. Process according to Claim 1 or 2,
**characterized in that**
the amount of the mixture of aquatic plants plus biogas booster in the fermentation mash of the biogas plant accounts in total for approximately 2% to 90% of the fresh weight of the fermentation mash of the thick phase introduced in the fermentation chamber.

4. Process according to one of the preceding claims,
**characterized in that**
the biogas plant is arranged in or directly next to the culture unit for aquatic, and that the heat circuits are connected together such that the culture unit is at least in part heated by the biogas plant's waste heat.

5. Process according to Claim 4,
**characterized in that**
the thick phase is pyrolyzed or treated in any other carbonization step.

6. Process according to one of the preceding claims,
**characterized in that,**
before being passed into the aquatic culture unit, the thin phase is aerated with atmospheric oxygen and/or provided with a precipitation agent, and first fed to a leguminous culture and/or Azolla culture for metabolizing the atmospheric oxygen generated, and then passed from there to the aquatic culture unit.

## Revendications

1. Procédé d'exploitation d'une installation de production de biogaz selon lequel du purin agricole et/ou des boues de fermentation déjà partiellement dégazées dans le cadre d'un processus de fermentation préalable et/ou des boues résiduaires et/ou des déchets issus de la production alimentaire sont mis à fermenter en tant que substrat de fermentation dans le cadre d'un autre processus de fermentation ou subissent une post-fermentation dans le cadre d'un autre processus de fermentation,
**caractérisé en ce que**
à partir du purin ou des boues de fermentation déjà partiellement dégazées ou des boues résiduaires ou des déchets issus de la production alimentaire, dans une première étape, la phase solide est séparée de la phase liquide, **en ce que** la phase solide est introduite dans la cuve de fermentation de l'installation de production de biogaz, **en ce que** la phase liquide séparée est acheminée jusqu'à un dispositif de culture contenant des plantes aquatiques à croissance rapide et au moins une partie de la biomasse issue des plantes aquatiques, qui peut être prélevée régulièrement du dispositif de culture, est acheminée jusqu'à la cuve de fermentation de l'installation de production de biogaz remplie de phase solide, et **en ce qu'**un booster de biogaz est incorporé dans la biomasse issue des plantes aquatiques avant ou lors de l'introduction dans la cuve de fermentation d'une installation de production de biogaz, le booster de biogaz étant constitué d'un ou de plusieurs esters d'acides gras ou d'une ou de plusieurs graisses ou huiles et/ou de drèches de distillerie et/ou de résidus contenant des graisses ou contenant des traces de sucres ou d'alcools ou des levures issus de la production alimentaire, ainsi que d'oligoéléments.

2. Procédé selon la revendication 1, **caractérisé en ce que** des lentilles d'eau ou des algues sont produites dans le dispositif de culture en tant que plantes aquatiques à croissance rapide.

3. Procédé selon la revendication 1 ou 2, **caractérisé en ce que** la proportion du mélange constitué par des plantes aquatiques plus le booster de biogaz dans les boues de fermentation de l'installation de production de biogaz est au total d'environ 2 % à 90 % de la masse humide des boues de fermentation de la phase solide introduite dans la cuve de fermentation.

4. Procédé selon l'une quelconque des revendications précédentes, **caractérisé en ce que** l'installation de biogaz est agencée dans ou directement à côté du dispositif de culture pour aquatiques, et **en ce que** les circuits de chaleur sont interconnectés de sorte que le dispositif de culture soit au moins partiellement chauffé par la chaleur résiduaire de l'installation de production de biogaz.

5. Procédé selon la revendication 4, **caractérisé en ce que** la phase solide est pyrolysée ou traitée dans une autre étape de carbonisation.

6. Procédé selon l'une quelconque des revendications précédentes, **caractérisé en ce que** la phase liquide est aérée avec de l'oxygène de l'air et/ou munie d'un agent de précipitation avant l'introduction dans le dispositif de culture aquatique, et tout d'abord introduite dans une culture de légumineuses et/ou d'azolla pour la métabolisation de l'azote de l'air formé, et ensuite acheminée depuis celle-ci dans le dispositif de culture aquatique.
